(19) Europäisches Patentamt
European Patent Office
Office européen des brevets

(11) Publication number: **0 087 835**
**B1**

# (12) EUROPEAN PATENT SPECIFICATION

(45) Date of publication of patent specification: **26.02.86**

(51) Int. Cl.[4]: **C 07 C 51/363,** C 07 C 53/19

(21) Application number: **83200258.8**

(22) Date of filing: **21.02.83**

(54) **Process for alpha halogenating alkyl carboxylic acids and catalyst composition for use therein.**

(30) Priority: **01.03.82 US 353285**

(43) Date of publication of application:
**07.09.83 Bulletin 83/36**

(45) Publication of the grant of the patent:
**26.02.86 Bulletin 86/09**

(84) Designated Contracting States:
**DE FR GB**

(56) References cited:
**EP-A-0 000 414**
**EP-A-0 057 291**
**US-A-4 368 140**

(73) Proprietor: **THE PROCTER & GAMBLE COMPANY**
**301 East Sixth Street**
**Cincinnati Ohio 45201 (US)**

(72) Inventor: **Gibson, Michael Steven**
**115 Main Street Apartment 2**
**Milford Ohio 45150 (US)**
Inventor: **Howie, John Keeney**
**1387 S. Nixon Camp Road**
**Oregonia Ohio 4054 (US)**

(74) Representative: **Gibson, Tony Nicholas et al**
**Procter & Gamble (NTC) Limited Whitley Road**
**Longbenton Newcastle upon Tyne NE12 9TS**
**(GB)**

**EP 0 087 835 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

FIELD OF THE INVENTION

The present invention relates to catalyst compositions and processes for halogenating $C_8$ to $C_{30}$ carboxylic acids at the alpha carbon atom.

BACKGROUND OF THE INVENTION

The halogenation of organic compounds at a specific carbon atom (i.e., regiospecifically) is difficult, especially on an industrial scale. In general, halogenation reactions tend to occur to some extent at all available carbon-hydrogen linkages in the molecule undergoing halogenation.

A successful process for achieving alpha-halogenation of carboxylic acids is described in U.S. Pat. No. 4,148,811, Crawford, issued April 10, 1979, corresponding to European Published Application No. 0000414. In that process, the reaction is carried out using a halogen source in the presence of a catalyst system comprising a cyanoquinone free radical inhibitor and an acidic material, preferably chlorosulfonic acid. Yields of 80—90°C of the alpha-halogenated carboxylic acid are achieved with the Crawford process.

Other references relating to alpha-halogenation of carboxylic acids (or their anhydrides or acid halides) are the following.

U.S. Pat. Nos. 3,751,461, Dhingra, issued August 7, 1973; 3,671,584, Schlecht, issued June 20, 1972; 4,007,222, Haschke et al., issued February 8, 1977; 3,584,036, Sexton, issued June 8, 1971; 3,634,504, Young, issued January 11, 1972; Harpp et al., J. Org. Chem., Vol. 40, No. 23,3420 (1975); Ogata et al., J. Org. Chem., Vol. 45, No. 14, 2831 (1980); Ogata et al., Can. J. Chem., Vol. 55, 1268 (1977); Ogata et al., Organic Synthesis, Vol. 59, 20 (1979); Ogata et al., Bull. Chem. Soc. Jap., Vol. 52, 255 (1979); Little et al., J. Am. Chem. Soc., Vol. 91, No. 25, 7098 (1969); Ogata et al., J. Org. Chem., Vol. 40, No. 20, 2960 (1975); Ogata et al., Nip. Kag. Kai, Vol. 9, 1517 (1975); Ogata et al., Jap. Pat. No. 135,024, October 25, 1975; Ogata et al., Tetrahedron, Vol. 26, 5929 (1970).

The alpha-halogenated carboxylic acids are useful in the production of lubricants and as surfactants and surfactant intermediates.

SUMMARY OF THE INVENTION

The present invention constitutes an improvement over the process of Crawford U.S. Pat. No. 4,148,811 in that higher yields are obtained.

In accordance with the present invention the alpha-halogenation of a $C_8$ to $C_{30}$ carboxylic acid is carried out with a halogen source in the presence of a catalyst system comprising a free radical inhibitor, a strong acid and an organic acid anhydride.

A representation of the reaction of the present invention is as follows:

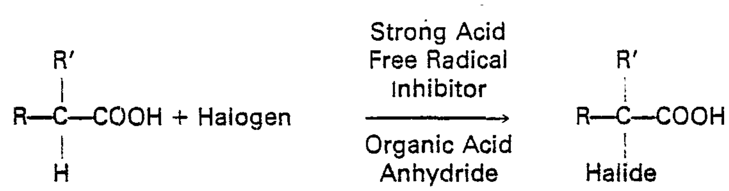


where R is a hydrocarbyl group and R′ is hydrogen or a hydrocarbyl group, the sum of the carbon atoms in R and R′ being from 6 to 28.

DETAILED DESCRIPTION OF THE INVENTION

The present invention encompasses a process for halogenating carboxylic acids which have at least one hydrogen atom on the carbon atom adjacent to the carboxylate group. The hydrogen atom is displaced by a halogen atom during the process.

In the practice of the invention, the carboxylic acid is contacted with halogen or a halogen source in the presence of an effective amount of a catalyst system comprising a free radical inhibitor, a strong acid and an organic acid anhydride.

By "halogenating" herein is meant displacing an alpha-hydrogen with halogens other than fluorine. As is well known, fluorination reactions are carried out under special conditions and are therefore not contemplated in the practice of this invention. The present process is particularly useful for chlorinating or brominating carboxylic acids, and on an industrial scale, is especially useful for chlorination reactions.

By an "effective amount" herein is meant an amount of the free radical inhibitor, strong acid and anhydride sufficient to direct the halogenation reaction regiospecifically such that it occurs almost exclusively at the alpha-hydrogen substituent of the carboxylic acid.

By "comprising" herein is meant that various other compatible materials may be present in the reaction mixtures during the halogenation reaction in such proportions as will not adversely affect the alpha-halogenation of the carboxylic acids. For example, various solvents and the like can optionally be present. The term "comprising" thus encompasses and includes the more restrictive terms "consisting of"

and "consisting essentially of" within its scope, so long as the processes and compositions of this invention include the specified ingredients, which are critical to the practice of the invention.

All percentages herein are on a mole basis, unless otherwise specified.

The carboxylic acids which are alpha-halogenated in the manner of this invention comprise those having from 8 to 30 carbon atoms and having at least one hydrogen on the carbon atom which is adjacent to the carboxyl group. The preferred carboxylic acids for use in the present process are alkyl carboxylic acids having 8 to 18 carbon atoms (also called fatty acids). Accordingly, the present process is especially useful for alpha-halogenation (preferably alpha-chlorination) of carboxylic acids such as lauric acid, myristic acid, palmitic acid, stearic acid and mixtures thereof.

The process of this invention is not limited with regard to the halogenation agent. Elemental halogens, liquid or gaseous, can be used. Chlorine gas is especially convenient, economical and preferred for use herein. Bromine can also be used, but is more expensive, as is iodine. Halogen sources other than elemental halogens can also be used. Such alternate halogen sources include, for example, well-known organic halogenating agents such as N-chlorosuccinimide (NCS) and N-bromosuccinimide (NBS).

Free Radical Inhibitors

A free radical inhibitor (also referred to in the art as "free radical scavenger") is an essential component of the catalyst system herein. The term "free radical inhibitor" is well known to those skilled in the art of organic reactions and many such materials are known. The following are exemplary.

A. Cyanoquinones

In general these compounds are characterized by the moiety

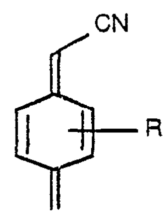

where R can be H or one or more substituent groups, e.g., halogen, alkoxyl, alkyl, thioalkyl, cyano, etc. See Wheland et al., *J. Am. Chem. Soc.* 98, 3916 (1976) and Wheland et al., *J. Org. Chem.* 40, 3101 (1975).

The tetracyanoquinodimethane (TCNQ) compounds preferred for use herein are of the formula (R as above):

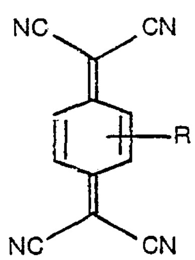

When R is hydrogen the compound is tetracyanoquinodimethane itself, which is the preferred free radical inhibitor herein.

Other specific examples of cyanoquinones useful herein include hexacyanobutadiene (HCBD) and tetracyanonaphthoquinodimethane (TNAP), represented by the following formulas:

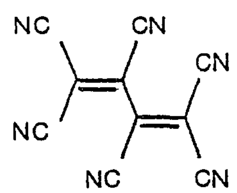

HCBD

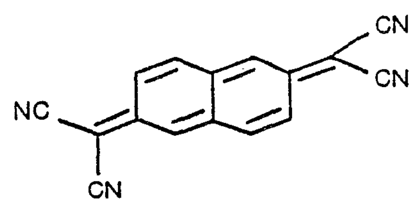

TNAP

TCNQ, TNAP and HCBD are prepared by techniques known in the literature. (While HCBD is not, in the most formal sense, a cyanoquinone structure, its extended conjugated system of electrons is "quinone-like". Accordingly, HCBD is considered a cyanoquinone in the present invention). See U.S. Pat. No. 4,148,811, noted above.

B. Quinones

These compounds contain the moiety

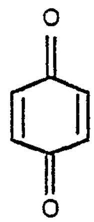

Examples of these compounds are benzoquinone, bromanil (tetrabromo-1,4-benzoquinone), chloranil (tetrachloro-1,4-benzoquinone), iodanil (tetraiodo--1,4-benzoquinone), 2,3,5-trichloro-1,4-benzoquinone and di- tri- and tetra cyano-1,4-benzoquinone.

C. Free radicals which are stable at room temperature (about 20°C).

These free radical compounds inhibit the formation of additional free radicals. An example of a stable free radical is diphenyl picrylhydrazil.

The preferred free radical inhibitors herein are the cyanoquinones, especially tetracyanoquinodimethane (TCNQ).

Other miscellaneous free radical inhibitors known to the art can also be used. Examples are elemental oxygen and m-dinitrobenzene.

Acids

The strong acids used in the catalyst system in the process herein include any of those known to the art for use in organic halogenation reactions. Such acidic materials include both Lewis acids and organic and inorganic protonic acids which are of greater strength than the carboxylic acids which are to be alpha-halogenated in the process herein. Typical examples of such acids include trifluoroacetic acid, $PBr_3$, $PCl_3$, thionyl chloride, $PCl_5$, phosgene, fluorosulfonic acid, chlorosulfonic acid, trifluoromethanesulfonic acid and sulfuric acid. The preferred acids are sulfuric acid and chlorosulfonic acid. The most preferred acid is sulfuric.

Organic Acid Anhydrides

The organic acid anhydrides used in the catalyst system in the process herein are organic compounds, which, upon reaction with water, yield an acid. Examples of these compounds are ketenes and carboxylic acid anhydrides. Ketenes contain the moiety $>C=C=O$ and carboxylic acid anhydrides contain the moiety

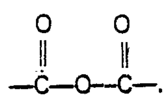.

If desired the organic acid anhydride can be the ketene or carboxylic anhydride of the fatty acid which is being halogenated. Preferably the ketene or carboxylic anhydride is that of a $C_2$ to $C_5$ carboxylic acid. The preferred acid anhydride is acetic acid anhydride.

The most preferred catalyst system herein is TCNQ:sulfuric acid:acetic anhydride.

The free radical inhibitor, strong acid and organic acid anhydride can be used in various ratios in the catalyst system herein. Typically the molar ratio of free radical inhibitor:strong acid will be from 0.001:1 to 100:1 (preferably 0.10:1 to 1.0:1), and the molar ratio of free radical inhibitor:organic acid anhydride will be from 0.001:1 to 100:1 (preferably 0.01:1 to 0.1:1).

The halogenation reaction of this invention is carried out by contacting the carboxylic acid with the halogen or halogen source in the presence of the free radical inhibitor/strong acid/organic acid anhydride catalyst at a temperature of 70°C, or greater. Chlorination reactions using elemental chlorine as the halogen are carried out at temperatures above 130°C, preferably at temperatures within the range from 145°C to 250°C. When halogen sources such as the N-halosuccinimides are used, temperatures of 70°C to 200°C are operative and convenient. Bromination and iodination reactions are carried out under similar temperature conditions.

The process herein can be carried out in the presence or absence of inert solvents. Preferably, the reaction is carried out without the use of solvents, and this is both convenient and economical on a commercial scale. Indeed, the use of solvents can lead to undesirable side reactions involving halogenation of many of the common hydrocarbon solvents. Under the reaction temperatures specified hereinabove, the carboxylic acids are liquids and are quite convenient to use in that state without additional solvents.

Typically the catalyst system is used at a concentration such that the molar concentration of the free radical inhibitor is from 0.01 to 10.0 molar percent, preferably 0.05 to 5 and most preferably 0.05 to 0.5 molar percent, relative to the amount of carboxylic acid.

The invention will be illustrated by the following nonlimiting example.

Example I

Preparation of 2-Chlorostearic Acid

A one-liter, three-necked round bottom reaction flask was mounted in a fume hood and placed in a 335 watt heating mantle. A mechanical stirrer, dry ice condenser and a fritted gas dispersion tube were fitted to the reaction flask. The temperature of the reaction flask was controlled by use of a Thermo-Watch which maintains the function of a Jack-O-Matic, upon which the heating mantle was placed. The dispersion tube was connected to a chlorine source by PVC tubing and metered with an in line flowmeter having a range of 0.05 to 1 liter/min.

The reaction vessel was initially charged with 1.0 mole of stearic acid (284.5 grams). The stearic acid was melted by raising the temperature to 80°C. At this time 0.005 mole TCNQ (1.02 grams) was added to the reaction vessel. Chlorine gas at a flow rate of 0.05 liter/min. was added to solubilize the TCNQ into the melted stearic acid. Acetic anhydride at a 0.06 molar level (6.125 grams) was then added to the reaction flask and the temperature was rapidly elevated to 130°C. Using a pipette, 0.016 mole sulfuric acid (1.6 grams) was then added to the reaction vessel. The temperature was then adjusted to 150°C and the chlorine flow rate was adjusted to 0.5 liter/min. This point was considered time zero for the beginning of the reaction.

After 52 minutes of reaction time, the chlorine flow rate was readjusted to 0.05 liter/min. and the reaction mixture was slowly cooled under a head of chlorine gas to the product melting point (64°C).

After cooling, the reaction product was analyzed by gas chromatography as being 96.12% 2-chlorostearic acid, 2.71% 2,2-dichlorostearic acid, 0.42% free radical chlorination products, and 0.75% unreacted stearic acid.

If the above reaction is carried out with fatty acids having alkyl chain lengths from 8 to 18 carbons, or combinations thereof, the corresponding 2-chloroalkyl acids are produced in purities exceeding 95%.

**Claims**

1. A process for alpha-halogenating alkyl carboxylic acids containing from 8 to 30 carbon atoms and having at least one reactive alpha-hydrogen substituent, comprising contacting said carboxylic acid with halogen or a halogen source in the presence of an effective amount of a catalyst system comprising:

A. An acid having a greater acid strength than the alkyl carboxylic acids;
B. A free radical inhibitor; and
C. An organic acid anhydride.

2. A process according to Claim 1 wherein the alpha-halogenation reaction is chlorination.

3. A process according to Claim 2 wherein the Component (B) free radical inhibitor is selected from cyanoquinones, quinones and free radicals which are stable at room temperature.

4. A process according to Claim 3 wherein the free radical inhibitor is a cyanoquinone of the formula

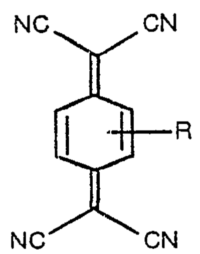

wherein R is a radical selected from hydrogen, halogen, alkoxyl, alkyl, thioalkyl and cyano.

5. A process according to Claim 3 wherein the free radical inhibitor is a quinone selected from tetrachloro-1,4-benzoquinone, 2,3,5-trichloro-1,4-benzoquinone and di-, tri-, and tetra-cyano-1,4-benzoquinone.

6. A process according to any of Claims 1—5 wherein the Component (A) strong acid is selected from trifluoroacetic acid, phosphorous trichloride, phosphorous tribromide, thionyl chloride, phosphorous pentachloride, phosgene, fluorosulfonic acid, chlorosulfonic acid, trifluoromethanesulfonic acid and sulfuric acid.

7. A process according to any one of Claims 1—6 wherein the Component (C) organic acid anhydride is selected from ketenes and carboxylic acid anhydrides having from 2 to 5 carbon atoms.

8. A process according to Claim 7 wherein the carboxylic acid is a $C_8$ to $C_{18}$ fatty acid, Component (A) is sulfuric acid, Component (B) is tetracyanoquinodimethane and Component (C) is acetic anhydride and the halogen source is elemental chlorine.

9. A process according to any one of Claims 1—8 wherein the molar ratio of (B):(A) in the catalyst system is from 0.001:1 to 100:1, and the molar ratio of (B):(C) is from 0.001:1 to 100:1.

10. A process according to any one of Claims 1—9 wherein the catalyst system is used at a level wherein the molar concentration of Component (B) is from 0.05% to 5% based on the amount of carboxylic acid.

11. A process according to any one of Claims 1—10 wherein the reaction temperature is from 145°C to 250°C.

12. A catalyst composition for use in the process of any one of Claims 1—11 comprising:

A. An acid having a greater acid strength than the $C_8$ to $C_{30}$ carboxylic acids;
B. A free radical inhibitor; and
C. An organic acid anhydride;

wherein the molar ratio of (B):(A) is from 0.10:1 to 1.0:1 and the molar ratio of (B):(C) is from 0.01:1 to 0.1:1.

**Patentansprüche**

1. Verfahren zur α-Halogenierung von Alkylcarbonsäuren mit 8 bis 30 Kohlenstoffatomen und mindestens einem reaktionsfähigen α-Wasserstoffsubstituenten, dadurch gekennzeichnet, daß die genannte Carbonsäure mit Halogen oder einer Halogenquelle in Gegenwart einer wirksamen Menge eines Katalysatorsystems umfassend:

A. eine Säure mit einer größeren Säurestärke als die Alkylcarbonsäuren;
B. einen Initiator freier Radikale; und
C. ein organisches Säureanhydrid

in Berührung gebracht wird.

2. Verfahren gemäß Anspruch 1, worin die α-Halogenierungsreaktion eine Chlorierung ist.

3. Verfahren gemäß Anspruch 2, worin der Bestandteil (B), der Initiator freier Radikale, ausgewählt ist aus Cycanochinonen, Chinonen und freien Radikalen, welche bei Raumtemperatur beständig sind.

4. Verfahren gemäß Anspruch 3, worin der Initiator freier Radikale ein Cyanochinon der Formel

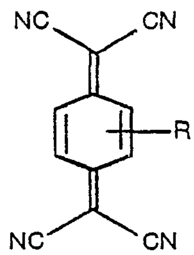

ist, worin R ein Rest ausgewählt aus Wasserstoff, Halogen, Alkoxyl, Alkyl, Thioalkyl und Cyano ist.

5. Verfahren gemäß Anspruch 3, worin der Initiator freier Radikale ein Chinon ausgewählt aus Tetrachlor-1,4-benzochinon, 2,3,5-Trichlor-1,4-benzochinon und Di-, Tri-, und Tetra-cyano-1,4-benzochinon ist.

6. Verfahren gemäß einem der Ansprüche 1 bis 5, worin der Bestandteil (A), die starke Säure, ausgewählt ist aus Trifluoressigsäure, Phosphortrichlorid, Phosphortribromid, Thionylchlorid, Phosphorpentachlorid, Phosgen, Fluorsulfonsäure, Chlorsulfonsäure, Trifluormethansulfonsäure und Schwefelsäure.

7. Verfahren gemäß einem der Ansprüche 1 bis 6, worin der Bestandteil (C), das organische Säureanhydrid, ausgewählt ist aus Ketenen und Carbonsäureanhydriden mit 2 bis 5 Kohlenstoffatomen.

8. Verfahren gemäß Anspruch 7, worin die Carbonsäure eine $C_8$- bis $C_{18}$-Fettsäure ist, der Bestandteil (A) Schwefelsäure ist, der Bestandteil (B) Tetracyanochinondimethan ist und der Bestandteil (C) Essigsäureanhydrid ist und die Halogenquelle elementares Chlor ist.

9. Verfahren gemäß einem der Ansprüche 1 bis 8, worin das Molverhältnis von (B) zu (A) in dem Katalysatorsystem 0,001:1 bis 100:1 beträgt und das Molverhältnis von (B) zu (C) 0,001:1 bis 100:1 beträgt.

10. Verfahren gemäß einem der Ansprüche 1 bis 9, worin das Katalysatorsystem in einer Menge verwendet wird, bei der die molare Konzentration des Bestandteils (B) 0,05 bis 5%, bezogen auf die Menge der Carbonsäure, beträgt.

11. Verfahren gemäß einem der Ansprüche 1 bis 10, worin die Reaktionstemperatur 145 bis 250°C beträgt.

12. Katalysatorzusammensetzung zur Verwendung in dem Verfahren nach einem der Ansprüche 1 bis 11, umfassend:

A. eine Säure mit einer größeren Säurestärke als die $C_8$ bis $C_{30}$-Carbonsäuren;
B. einen Initator freier Radikale; und
C. ein organisches Säureanhydrid;

worin das Molverhältnis von (B) zu (A) 0,10:1 bis 1,0:1 beträgt und das Molverhältnis von (B) zu (C) 0,01:1 bis 0,1:1 beträgt.

**Revendications**

1. Procédé d'halogénation en position alpha d'acides alkylcarboxyliques contenant de 8 à 30 atomes de carbone et ayant au moins un substituant hydrogène en position alpha réactif, comprenant la mise en contact du dit acide carboxylique avec un halogène ou une source d'halogène en présence d'une quantité efficace d'un système catalytique comprenant:

A. Un acide ayant une force acide supérieure à celle des acides alkylcarboxyliques;
B. Un inhibiteur de radicaux libres; et
C. Un anhydride d'acide organique.

2. Procédé selon la revendication 1, dans lequel la réaction d'halogénation en position alpha est une chloration.

3. Procédé selon la revendication 2, dans laquelle le constituant inhibiteur de radicaux libres (B) est choisi parmi les cyanoquinones, les quinones et les radicaux libres qui sont stables à la température ambiante.

4. Procédé selon la revendication 3, dans lequel l'inhibiteur de radicaux libres est une cyanoquinone de formule

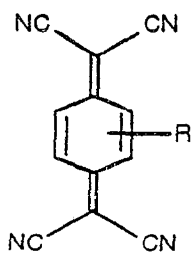

dans laquelle R est un radical choisi parmi l'hydrogène, un halogène, un groupe alcoxy, alkyle, thioalkyle et cyano.

5. Procédé selon la revendication 3, dans lequel l'inhibiteur de radicaux libres est une quinone choisie parmi la tétrachloro-1,4-benzoquinone, la 2,3,5-trichloro-1,4-benzoquinone et les di-, tri- et tétra-cyano-1,4-benzoquinone.

6. Procédé selon l'une quelconque des revendications 1 à 5, dans lequel le constituant acide fort (A) est choisi parmi l'acide trifluoroacétique, le trichlorure de phosphore, le tribromure de phosphore, le chlorure de thionyle, le pentachlorure de phosphore, le phosgène, l'acide fluorosulfonique, l'acide chlorosulfonique, l'acide trifluorométhane-sulfonique et l'acide sulfurique.

7. Procédé selon l'une quelconque des revendications 1 à 6, dans lequel le constituant anhydride d'acide organique (C) est choisi parmi les cétènes et les anhydrides d'acide carboxylique ayant de 2 à 5 atomes de carbone.

8. Procédé selon la revendication 7, dans lequel l'acide carboxylique est un acide gras en $C_8$ à $C_{18}$, le constituant (A) est l'acide sulfurique, le constituant (B) est le tétracyanoquinodiméthane et le constituant (C) est l'anhydride acétique et la source d'halogène est le chlore élémentaire.

9. Procédé selon l'une quelconque des revendications 1 à 8, dans lequel la proportion molaire de (B):(A) dans le système catalytique est de 0,001:1 à 100:1 et la proportion molaire de (B):(C) est de 0,001:1 à 100:1.

10. Procédé selon l'une quelconque des revendications 1 à 9, dans lequel le système catalytique est utilisé en proportion telle que la concentration molaire du constituant (B) soit de 0,05% à 5%, par rapport à la quantité d'acide carboxylique.

11. Procédé selon l'une quelconque des revendications 1 à 10, dans lequel la température de la réaction est de 145°C à 250°C.

12. Composition catalytique utilisable dans le procédé de l'une quelconque des revendications 1 à 11, comprenant:

A. Un acide ayant une force acide supérieure à celle des acides carboxyliques en $C_8$ à $C_{30}$;
B. Un inhibiteur de radicaux libres; et
C. Un anhydride d'acide organique;

dans laquelle la proportion molaire de (B):(A) est de 0,10:1 à 1,0:1 et la proportion molaire de (B):(C) est de 0,01:1 à 0,1:1.